(19) Europäisches Patentamt
European Patent Office
Office européen des brevets

(11) Numéro de publication: **0 240 422 B1**

(12) **FASCICULE DE BREVET EUROPEEN**

(45) Date de publication du fascicule du brevet: **29.08.90**

(51) Int. Cl.[5]: **C07C 229/22**, C12P 13/00

(21) Numéro de dépôt: **87400692.7**

(22) Date de dépôt: **27.03.87**

(54) **Procédé de purification de la carnitine.**

(30) Priorité: **04.04.86 FR 8604832**

(43) Date de publication de la demande:
**07.10.87 Bulletin 87/41**

(45) Mention de la délivrance du brevet:
**29.08.90 Bulletin 90/35**

(84) Etats contractants désignés:
**AT BE CH DE GB IT LI LU**

(56) Documents cités:
**Néant**

(73) Titulaire: **SOCIETE NATIONALE ELF AQUITAINE Société anonyme dite, Tour Elf 2, Place de la Coupole La Défense 6, F-92400 Courbevoie(FR)**

(72) Inventeur: **Souppe, Jérôme, 3 rue de Babsalle, F-64000 Pau(FR)**
Inventeur: **Haurat, Gisèle, Quartier Bergoue, F-64370 Arthez-de-Béarn(FR)**
Inventeur: **Goulas, Philippe, 3 Place Mulot, F-64000 Pau(FR)**

(74) Mandataire: **Kohn, Armand, 5 Avenue Foch, F-92380 Garches(FR)**

EP 0 240 422 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne un nouveau procédé pour la purification de la carnitine, c'est-à-dire de l'acide triméthylamino-4 hydroxy-3 butyrique, et en particulier de la L-carnitine obtenue par voie biochimique.

La carnitine étant principalement employée pour des applications biologiques, notamment pharmacologiques, cosmétiques et alimentaires,sa pureté est une condition importante d'emploi. D'autre part, comme les méthodes chimiques de sa préparation conduisent à des mélanges racémiques, alors que seul le stéréoisomère L est biologiquement actif, c'est surtout le procédé biochemique de préparation qui est intéressant, parce qu'il fournit l'isomère L. Il est donc important de disposer d'un procédé de purification permettant d'éliminer convenablement les impuretés qu'apportent les matières utilisées dans la voie biochimique, en particulier différentes protéines et substances minérales. C'est l'avantage intéressant qu'apporte le procédé suivant l'invention ; il permet d'éviter la méthode classique de chromatcgraphie qui n'élimine pas les composés minéraux.

Ce nouveau procédé, suivant l'invention, rend possible l'obtention directe de la carnitine sous sa forme amphotère au lieu d'un sel d'acide fort, en particulier chlorhydrate, comme c'est le cas dans la technique antérieure, par exemple dans le brevet FR-2 398 046. D'autre part, il ne comporte qu'au maximum 5 étapes, alors qu'il y a au moins 10 opérations successives dans le procédé connu, utilisant une colonne à résine échangeuse d'ions.

Le procédé de purification suivant l'invention est caractérisé en ce que d'abord la milieu aqueux, renfermant la carnitine, est acidifié, de façon à faire précipiter les protéines présentes, et, après la séparation de celles-ci, l'eau du milieu est remplacée par un alcool inférieur, dissolvant au moins à chaud la carnitine, tandis que les substances minérales précipitent et se séparent de la solution. On obtient ainsi une solution alcoolique riche en sel de carnitine de bonne pureté.

Lorsqu'un produit encore plus pur est désiré et, notamment, quand l'obtention de composé

$$(CH_3)_3\overset{+}{N}-CH_2\underset{\displaystyle OH}{\underset{|}{C}H}CH_2COO^-$$

amphotère, lui-même et non de son sel,est visée, on complète la purification de la façon suivante. Le solide, extrait de la solution alcoolique susmentionnée, est remis en solution aqueuse que l'on neutralise avec une base jusqu'à environ 7,2, et la solution neutralisée est traitée en sorte que l'eau y soit remplacée par un alcool inférieur, ce qui fait dissoudre la carnitine et précipiter le sel de la base de neutralisation de l'acide qui a servi à l'acidification du milieu initial. La solution alcoolique restante contient la carnitine amphotère d'excellente pureté, qui peut être récupérée à l'état solide, notamment par cristallisation à froid, par dessication de la solution ou par précipitation par addition d'un liquide non solvant.

Voici, plus en détail, cing étapes du procédé suivant l'invention, susceptibles de fournir une L-carnitine très pure à partir d'une solution aqueuse impure de ce composé,en particulier provenant du milieu issu de la bioconversion de déhydro-3 carnitine.

### 1°- ACIDIFICATION DU MILIEU

Un acide, de préférence fort, est ajouté à la solution aqueuse, de façon à abaisser le pH de celle-ci vers une valeur d'environ 1 à 3, de préférence voisine ou égale à 2. Des acides tels que sulfurique, sulfureux, phosphorique, perchlorique, benzène-sulfonique, etc. peuvent être employés, le $SO_4H_2$ convenant particulièrement bien.

Cette opération a pour effet la précipitation des protéines qui se trouvaient dans le milieu traité, et qui - ainsi - peuvent être éliminées par filtration ou centrifugation du liquide acidifié.

### 2°- PRECIPITATION DE SELS MINERAUX

Le filtrat provenant de l'opération précédente est traité de façon à remplacer son eau par un alcool inférieur, capable de dissoudre la carnitine. Cela peut être réalisé par l'évaporation de l'eau de la solution et addition de l'alcool, ou bien par évaporation avec entraînement à l'alcool. A la fin, la solution alcoolique est maintenue à la température à laquelle la carnitine est toute dissoute, alors que les composés minéraux précipitent et on les sépare. L'alcool peut être tel que méthanol,étahnol, méthoxyéthanol, isopropanol, n-propanol, n-butanol, tert-butanol, ou isobutanol, l'éthanol étant particulièrement approprié. Des mélanges d'alcools peuvent être employées. Selon la nature de l'alcool et la solubilité de la carnitine, on opère à une température appropriée, qui est en général de 20° à 100°C.

### 3°- REMISE EN SOLUTION AQUEUSE

A partir de la solution alcoolique, obtenue au stade précédent, 2°-, on peut récupérer la carnitine dissoute, sous la forme d'un sel de l'acide employé au stade 1°-, à l'état de bonne pureté. Mais si l'on cherche à pousser la purification encore plus loin, ou s'il s'agit de produire la carnitine amphotère au lieu de

son sel, on procède comme suit. De la solution alcoolique on extrait le sel de carnitine, notamment par évaporation de l'alcool ou par précipitation au moyen d'un non solvant, tel que - par exemple - une cétone. Ce dernier moyen contribue encore à améliorer la pu reté. On dissout ce sel dans l'eau, de préférence à raison de 100 à 500 g par litre, et on soumet la solution au stade 4°- suivant.

4°- NEUTRALISATION

La solution, issue de l'étape 3°- précédente, est neutralisée par l'adjonction d'une base, le plus souvent soude. Bien que le pH en fin de neutralisation puisse osciller entre 6,9 et 7,4, il est de préférence très proche ou égal à 7,2. Cette neutralisation libère la carnitine amphotère suivant la réaction :

$$HSO_4^- . (CH_3)_3 NCH2\underset{\displaystyle OH}{C}HCH_2COOH + 2Na^+.OH^-$$

$$\longrightarrow (CH_3)_3\overset{+}{N}CH_2\underset{\displaystyle OH}{C}HCH_2CO\bar{O} \quad + \quad Na_2SO_4 \quad + 2H_2O$$

en prenant, à titre d'exemple, l'acide sulfurique, pour l'acidification suivant 1°-, et la soude en tant que base de neutralisation.

La solution, ainsi neutralisée, est traitée en vue de remplacement de son eau par un alcool inférieur, capable de dissoudre la carnitine. Cela peut être opéré comme sous 2°- plus haut, c'est-à-dire par évaporation de l'eau, de préférence sous vide, et addition de l'alcool, ou bien par évaporation sous vide avec entraînement à l'alcool.

La nouvelle solution alcoolique, obtenue de cette manière, est maintenue à la température à laquelle la carnitine reste dissoute, alors que le sel minéral (sulfate de Na dans l'exemple ci-dessus), formé du fait de la neutralisation, précipite.

On dispose donc d'une solution alcoolique de carnitine très pure.

5°- RECUPERATION A L'ETAT SEC

A partir de la solution alcoolique restant après 4°-, on peut récupérer la carnitine solide, sèche, par évaporation complète du solvant. Un autre moyen qui parfait encore la pureté consiste à précipiter la carnitine par l'adjonction d'un non solvant à sa solution alcoolique ; ainsi l'addition d'un excès d'acétone permet-elle de faire déposer de la carnitine solide, très pure.

Comme mentionné plus haut, bien que tout échantillon de carnitine, quelle que soit son origine, puisse être purifié par le procédé de la présente invention, celle-ci s'applique particulièrement bien aux milieux issus de la bioconversion de la déhydrocarnitine par réduction asymétrique de celle-ci à l'aide de NADH, avec réduction du NAD+ formé par l'ion formiate, en présence de formiate déshydrogénase (FDH). Les rendements en L-carnitine et la purification sont remarquables lorsque l'hydrogénase FDH provient des levures Candida bodiini ou Pichia pastoris.

C'est à ces derniers types de solutions de carnitine qu'ont trait les exemples non limitatifs donnés ci-après.

EXEMPLE 1

La préparation de la L-carnitine est effectuée par l'action du nicotinamide adénine dinucléotide réduit, NADH, sur la déhydrocarnitine, et réduction du NAD+ formé, au moyen du groupe formique avec de la formiate déshydrogénase FDH comme catalyseur.

Le schéma de synthèse est donc :

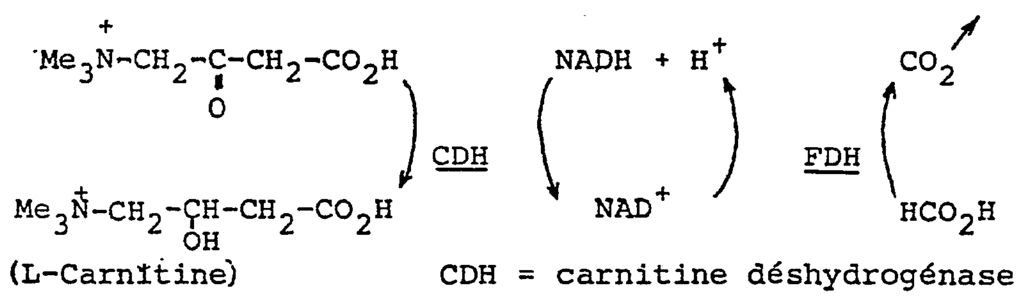


(L-Carnitine) CDH = carnitine déshydrogénase

La CDH utilisée provenait d'un extrait de culture de Pseudomona putida. Elle était préciptée au sulfate d'ammonium à 250 g par litre d'extrait brut (55% de la saturation). Le culot, obtenu à partir de 20 ml d'extrait, était redissous dans 7 ml de tampon de $Na_2HPO_4/KH_2PO_4$ 50mM de pH-7,0, la solution passée sur gel filtration (G200), puis chromatographiée sur 5'AMP - Sépahrose 4B (enzyme fixée par affinité puis éluée avec $NAD^+$ 10 mM) et enfin passée sur gel filtration ACA 44 pour récupérer l'enzyme dépourvue de $NAD^+$ et du $NaN_3$(lequel avait été mis dans les tampons d'élution pour se protéger des contaminations).

L'enzyme pure, ainsi obtenue, avait une activité spécifique de 55 U/mg ; on en a introduit 40 U dans un réacteur de 50 ml.

Ce réacteur contenait en outre : 2mM de $NAD^+$, 150 mM de $HCO_2SNH_4$, 6 mg de chloramphénicol et 40 U soit 60 mg de lyophilisat commercial de formiate déshydrogénase, FDH, provenant de la Candida bodiini (EC 1.2.1.2. -- commercialisée par BOEHRINGER MANNHEIM).

Le réacteur était alimenté par une solution maintenue à 4°C, contenant du chlorhydrate de déhydrocarnitine cristallisé, dissous à la concentration 0,8 M, ainsi que de l'acide formique à 0,8 M. Le débit d'injection était de 1,2 ml/h. La température dans le réacteur était réglée à 30°C et le pH maintenu à 7,0 par addition d'ammoniaque 2 N contrôlée par un système de régulation de pH.

Après 230 h on a atteint un volume final de 315 ml et une concentration en L-Carnitine de 477 mM correspondant à un rendement de 98%.

La solution, ainsi obtenue, est soumise aux opérations de purification suivantes.

Ce milieu de bioconversion est acidifié à pH : 2,0 par de l'acide sulfurique concentré. Les protéines précipitent. Après filtration, le filtrat est évaporé sous vide (70°C/15 mmHg) suivi d'un entraînement de l'eau à l'éthanol. Le milieu s'appauvrit en eau et s'enrichit en éthanol. La L-carnitine est soluble dans l'éthanol à chaud ; les sels minéraux ne le sont pas, et ils précipitent. Après filtrations sur fritté, on récupère une solution éthanolique de sulfate de L-Carnitine que l'on précipite à l'acétone à froid (rapport volumétrique : acétone/éthanol = 90/10). Le sulfate de L-Carnitine est dissous dans l'eau et neutralisé à pH=7,2 par de la soude concentrée.

La solution aqueuse obtenue est évaporée sous vide avec entraînement à l'éthanol. Le milieu s'appauvrit en eau et le sulfate de sodium précipite dans l'éthanol. Après filtration sur fritté, on récupère une solution éthanolique de L-Carnitine qui ne contient aucun sulfate (test au $Ba^{2+}$). On évapore le solvant jusqu'à obtention d'un produit sec. Ce dernier est dissous dans un minimum d'éthanol à chaud (40°C) et précipté à froid à l'acétone. Le rendement global de la purification est de 80%. Le rendement de l'ensemble de la préparation en L-carnitine pure ressort donc à 80x98/100=78,4%.

Les caractéristiques du produit obtenu sont :

. $[\alpha]_D^{20}$ = -30,2° (2,5,eau)

. sulfates < 400 ppm

. $^1H$ RMN ($D_2O$) (300 MHz) : δ (ppm) :

4,4(quintuplet ; J:5Hz ; 1H);3,25(doublet) J:5Hz;2H) ; 3,05(singulet,9H);2,25(doublet de doublet;2H).

. Dosage enzymatique : 100% L-carnitine.

EXEMPLE 2 (comparatif)

Toutes les opérations de l'exemple 1 sont répétées avec la seule différence que la FDH employée provenait de la levure Torulopsis candida (GFP 206) comme décrit dans l'exemple 5, page 8, du brevet français 2398046. Le rendement en L-carnitine purifiée était alors de 58% seulement, et la pureté du produit final n'était que de 92%. Cela montre l'intérêt de la FDH provenant de la Candida bodiini, correspondant à la forme préférée de la présente invention.

EXEMPLE 3

Cherchant à limiter l'augmentation de volume au cours de la bioconversion, on a mis en oeuvre des réactifs en solutions plus concentrées en déhydrocarnitine, acide formique et ammoniaque. D'autre part, on a réduit la concentration en $NAD^+$.

Ainsi a-t-on chargé dans le réacteur 50 ml de solution contenant :

$Na_2HO_4/KH_2PO_4$ 50 mM pH : 7,5

$NAD^+$ 0,2 mM

6 mg chloramphénicol

40U de CDH sommairement purifiée comme décrit ci-après

25U de FDH BOEHRINGER-MANNHEIN provenant de la Candida bodiini.

La CDH, extraite de P. Putida, avait été précipitée au sulfate d'ammonium (55% de la saturation), centrifugée et le culot redissous dans le tampon phosphate 50mM pH : 7,5 ; la solution obtenue dialysée pendant 16 h contre ce même tampon.

Le réacteur était alimenté avec une solution maintenue à 4°C, contenant la déhydrocarnitine et l'acide formique à la concentration, l'un et l'autre, de 1,6 M. Le débit était de 0,6 ml/h. La température du réacteur maintenue à 30°C et le pH 7,5 par addition contrôlée d'ammoniaque 8N. Après 48 heures, on atteint

un volume final de 90 ml et une concentration en L-Carnitine de 500 mM, soit un rendement de 98%.

La purification effectuée comme dans l'exemple 1, donne le même résultat.

EXEMPLE 4

Le même essai que dans l'exemple 3 a été mené avec de la FDH de Pichia Pastoris avec les modifications suivantes :

$NAD^+$ 0,4 mM

$HCO_2NH_4$ 500 mM

absence de chloramphénicol qui a été omis, parce que la forte salinité dûe à la concentration initiale élevée en formiate d'ammonium limite les risques de contamination. FDH extraite d'une culture de Pichia Pastoris précipitée au sulfate d'ammonium (50% de la saturation), centrifugée et le culot redissous dans du tampon phosphate 50 mM pH 7,5, la solution obtenue dialysée 16 heures contre ce tampon.

Les autres paramètres du réacteur étaient ceux de l'exemple 3.

Après 49 h le volume final est de 60 ml et la concentration en L-carnitine de 700 mM ce qui correspond à un rendement de 93%.

Le dosage des enzymes a montré une activité résiduelle de 90% en CDH et de 90% en FDH.

La purification de la L-carnitine obtenue, effectuée de la même façon qu'à l'exemple 1, a donné le même résultat : il en résulte que la FDH de la Pichia pastoris donne des résultats équivalents à ceux de la FDH de Candida bodiini, bien meilleurs des résultats obtenus avec la FDH de Torulopsis candida (exemple 2).

**Revendications**

1. Procédé de purification de la carnitine, caractérisé en ce que d'abord le milieu aqueux,renfermant la carnitine, est acidifié, de façon à faire précipiter les protéines présentes, et, après la séparation de celles-ci, l'eau du milieu est remplacée par un alcool inférieur, dissolvant la carnitine, au moins à chaud, tandis que les substances minérales précipitent et se séparent de la solution.

2. Procédé suivant la revendication 1,caractérisé en ce que le solide, extrait de la solution alcoolique obtenue, est remis en solution aqueuse que l'on neutralise avec une base jusqu'à un pH compris entre 6,9 et 7,4, et la solution neutralisée est traitée en sorte que l'eau y soit remplacée par un alcool inférieur, de façon à y dissoudre la carnitine et à précipiter le sel de la base de neutralisation avec l'acide qui a servi à l'acidification du milieu initial.

3. Procédé suivant la revendication 1 ou 2, caractérisé en ce que l'acidification est effectuée de manière à amener le pH du milieu à une valeur de 1 à 3, de préférence à 2 ou au voisinage de 2.

4. Procédé suivant une des revendications 1 à 3, caractérisé en ce que l'acide, utilisé pour l'acidification, est sulfureux, phosphorique,perchlorique ou benzène-sulfonique, et de préférence sulfurique.

5. Procédé suivant une des revendications précédentes, caractérisé en ce que l'alcool inférieur, employé, est le méthanol, éthanol, isopropanol, n-propanol, n-butanol, isobutanol, tert-butanol ou un mélange de tels alcools, l'éthanol étant préféré.

6. Procédé suivant une des revendication précédentes, caractérisé en ce que la séparation de composés minéraux au sein de l'alcool a lieu à une température comprise entre 20° et 100°C, à laquelle la carnitine est complètement dissoute.

7.Procédé suivant une des revendications précédentes, caractérisé en ce qu'après la séparation des composés minéraux, la carnitine est remise en solution aqueuse, de préférence à raison de 100 à 500 g par litre.

8. Procédé suivant la revendication 7, caractérisé en ce qu'une base est ajoutée à la solution aqueuse, de façon à libérer la carnitine amphotère, après quoi l'eau de la solution est remplacée par un alcool inférieur, de façon à faire précipiter le sel minéral formé par l'addition de la base ; et la solution alcoolique, renfermant la carnitine amphotère purifiée, est récupérée.

**Claims**

1. Process of purification of carnitine, characterised in that the aqueous medium containing the carnitine is first acidified, in order to precipitate the proteins present and after their separation the water of the medium is replaced with a lower alcohol dissolving carnitine at least in the hot, while the mineral substances precipitate and separate from the solution.

2. Process according to claim 1, characterised in that the solid extracted from the alcoholic solution obtained is taken up into aqueous solution which is neutralised with a base to pH between 6.9 to 7.4 and neutralised solution is treated so as to replace its water with a lower alcohol, in order to dissolve the carnitine and precipitate the salt of the base of neutralisation with the acid which has been used for acidification of the initial medium.

3. Process according to claim 1 or 2, characterised in that the acidification is effected in a manner so that the pH of the medium has a value from 1 to 3, preferably 2 or about 2.

4. Process according to any of claims 1 to 3, characterised in that the acid used for the acidification is sulphurous, phosphoric, perchloric or benzenesulphonic and preferably sulphuric.

5. Process according to any of the preceding claims, characterised in that the lower alcohol employed is menthol, ethanol, isopropanol, n-propanol, n-butanol, isobutanol, tert.-butanol or a mixture of such alcohols, ethanol being preferred.

6. Process according to any of the preceding claims, characterised in that separation of the mineral compounds from the alcohol takes place at a temperature in the range from 20° to 100°C at which the carnitine is completely dissolved.

7. Process according to any of the preceding claims, characterised in that after separation of the mineral compounds the carnitine is taken up in aqueous solution, preferably at the concentration of guerillas to 500 g/l.

8. Process according to claim 7, characterised in that a base is added to the aqueous solution in order to liberate amphoteric carnitine, after which the water of the solution is replaced with a lower alcohol, so as to precipitate the mineral salt formed by addition of the base, the alcoholic solution containing the purified amphoteric carnitine being recovered.

**Patentansprüche**

1. Verfahren zur Reinigung von Carnitin, dadurch gekennzeichnet, dass zunächst das das Carnitin enthaltende wässerige Medium angesäuert wird, um die vorhandenen Proteine auszufällen, und nach der Abtrennung derselben das Wasser im Medium durch einen niederen Alkohol ersetzt wird, wobei zumindest in der Wärme das Carnitin gelöst wird, während die mineralischen Substanzen ausfällen und sich aus der Lösung abscheiden.

2. Verfahren nach Anspruch 1, dadurch gekennzeichnet, dass der aus der erhaltenen alkoholischen Lösung extrahierte Feststoff wieder in wässerige Lösung gebracht wird, die dann mit einer Base bis zu einem pH-Wert zwischen 6,9 und 7,4 neutralisiert wird, und die neutralisierte Lösung so behandelt wird, dass das Wasser darin durch einen niederen Alkohol ersetzt wird, um das Carnitin zu lösen und das Salz der zur Neutralisation verwendeten Base mit der Säure, die zur Ansäuerung des anfänglichen Mediums gedient hat, auszufällen.

3. Verfahren nach Anspruch 1 oder 2, dadurch gekennzeichnet, dass die Ansäuerung so durchgeführt wird, dass der pH-Wert des Mediums auf 1 bis 3, vorzugsweise 2 oder in der Nähe von 2 eingestellt wird.

4. Verfahren nach einem der Ansprüche 1 bis 3, dadurch gekennzeichnet, dass es sich bei der zur Ansäuerung verwendeten Säure um schweflige Säure, Phosphorsäure, Perchlorsäure oder Benzolsulfonsäure und vorzugsweise Schwefelsäure handelt.

5. Verfahren nach einem der vorstehenden Ansprüche, dadurch gekennzeichnet, dass es sich beim verwendeten niederen Alkohol um Methanol, Ethanol, Isopropanol, n-Propanol, n-Butanol, Isobutanol, tert-Butanol oder um ein Gemisch derartiger Alkohole handelt, wobei Ethanol bevorzugt ist.

6. Verfahren nach einem der vorstehenden Ansprüche, dadurch gekennzeichnet, dass die Abtrennung der mineralischen Verbindungen vom Alkohol bei einer Temperatur von 20 bis 100°C stattfindet, bei der das Carnitin vollständig gelöst ist.

7. Verfahren nach einem der vorstehenden Ansprüche, dadurch gekennzeichnet, dass nach der Abtrennung der mineralischen Verbindungen das Carnitin wieder in wässerige Lösung vorzugsweise mit einem Gehalt an 100 bis 500 g pro Liter übergeführt wird.

8. Verfahren nach Anspruch 7, dadurch gekennzeichnet, dass die wässerige Lösung mit einer Base versetzt wird, um das amphotere Carnitin freizusetzen, wonach das Wasser der Lösung durch einen niederen Alkohol ersetzt wird, um das durch die Zugabe der Base gebildete Mineralsalz auszufällen, und die das gereinigte amphotere Carnitin enthaltende alkoholische Lösung gewonnen wird.